# EUROPÄISCHE PATENTANMELDUNG

(11) **EP 4 813 307 A1**
(43) Veröffentlichungstag der Anmeldung: **30.09.2026**
(21) Anmeldenummer: 25167054.3
(22) Anmeldetag: 28.03.2025
(51) Int. Cl.: A61B 6/02, A61B 6/03, A61B 6/00

(54) **COMPUTERIMPLEMENTIERTES MAA-VERFAHREN ZUM BETRIEB EINER RÖNTGENEINRICHTUNG, RÖNTGENEINRICHTUNG, COMPUTERPROGRAMM UND ELEKTRONISCH LESBARER DATENTRÄGER**

(71) Anmelder: Siemens Healthineers AG, 91301 Forchheim (DE); Friedrich-Alexander-Universität Erlangen-Nürnberg, in Vertretung des Freistaates Bayern, 91058 Erlangen (DE)
(72) Erfinder: Kreher, Björn, 91094 Bräuningshof (DE); Rohleder, Maximilian, 91052 Erlangen (DE)

(57) **Zusammenfassung**

Die Erfindung betrifft ein computerimplementiertes Verfahren zum Betrieb einer Röntgeneinrichtung (12), die zur Aufnahme von zweidimensionalen Projektionsbildern in verschiedenen Projektionsgeometrien entlang einer Aufnahmetrajektorie, aus denen ein dreidimensionaler Bilddatensatz rekonstruierbar ist, eine Aufnahmeanordnung mit einem Röntgenstrahler (14) und einem Röntgendetektor (15) umfasst, wobei vor der Aufnahme eines Aufnahmebereichs, der wenigstens ein artefaktrelevantes Metallobjekt (2) umfasst, zur Ermittlung einer artefaktreduzierenden Aufnahmetrajektorie
- eine Objektinformation, die die dreidimensionale Position, Orientierung und Ausdehnung für jedes des wenigstens einen artefaktrelevanten Metallobjekts (2) beschreibt, ermittelt wird,
- aus der Objektinformation für jedes artefaktrelevante Metallobjekt (2) ein Objektmodell, das wenigstens eine dreidimensionale geometrische Grundform, die das artefaktrelevante Metallobjekt (2) beschreibt, und deren Position, Größe und Orientierung im Aufnahmebereich beschreibt, ermittelt wird, und
- eine Artefaktmaßinformation, die die Stärke auftretender Metallartefakte beschreibt, für wenigstens eines des wenigstens einen artefaktrelevanten Metallobjekts (2) und wenigstens eine Projektionsgeometrie unter Verwendung wenigstens eines des wenigstens einen Objektmodells ermittelt wird, wobei die Artefaktmaßinformation bei der Ermittlung der artefaktreduzierenden Aufnahmetrajektorie verwendet wird.

## Beschreibung

Die Erfindung betrifft ein computerimplementiertes Verfahren zum Betrieb einer Röntgeneinrichtung, die zur Aufnahme von zweidimensionalen Projektionsbildern in verschiedenen Projektionsgeometrien entlang einer Aufnahmetrajektorie, aus denen ein dreidimensionaler Bilddatensatz rekonstruierbar ist, eine Aufnahmeanordnung mit einem Röntgenstrahler und einem Röntgendetektor umfasst, wobei vor der Aufnahme eines Aufnahmebereichs, der wenigstens ein artefaktrelevantes Metallobjekt umfasst, eine artefaktreduzierende Aufnahmetrajektorie ermittelt wird. Daneben betrifft die Erfindung eine Röntgeneinrichtung, ein Computerprogramm und einen elektronisch lesbaren Datenträger.

Im Stand der Technik ist es bekannt, Computertomographie auch mit nicht dedizierten Röntgeneinrichtungen durchzuführen. Beispielsweise ist es bekannt, bei einer Röntgeneinrichtung mit einem C-Bogen die Aufnahmetrajektorie zur Aufnahme zweidimensionaler Projektionsbilder mittels des C-Bogens, an dem sich gegenüberliegend der Röntgenstrahler und der Röntgendetektor anordnet sind, zu realisieren. Nachdem bei derartigen Vorgehensweisen üblicherweise eine Kegelstrahlgeometrie des Strahlenfeldes genutzt wird, ist auch von Kegelstrahl-Computertomographie (Cone Beam CT - CBCT) die Rede. Beispielsweise können dann vorbereitend, unterstützend und/oder nachbereitend und für medizinische Interventionen Bildaufnahmen erfolgen.

Ein Problem sind durch Metallobjekte im Aufnahmebereich entstehende Artefakte. Dies ist insbesondere dann relevant, wenn die Metallobjekte selbst und/oder unmittelbar benachbarte anatomische Bereiche Gegenstand bzw. Ziel der Bildgebung sind. Beispielsweise ist es bekannt, beim Setzen von Implantaten die Positionierung der Implantate durch CBCT zu überprüfen. Ein konkretes Beispiel ist das Setzen von Pedikelschrauben. Bei solchen Anwendungen verschleiern Metallartefakten klinisch relevante anatomische Details nahe an dem Metallobjekt, sodass auch die klinische Nutzbarkeit von CBCT-Bilddatensätzen reduziert würde.

Daher wurde im Stand der Technik bereits vorgeschlagen, Verfahren zur Reduzierung von Metallartefakten in der Nachverarbeitung (Metal Artifact Reduction, MAR) und/oder zur Vermeidung von starken Metallartefakten während der Datenaufnahme (Metal Artifact Avoidance, MAA) anzuwenden.

Beispielsweise sind für die MAR sogenannte Inpainting-Methoden bekannt, bei denen zunächst Metall enthaltende Voxel (Metallvoxel) in einer ersten Rekonstruktion segmentiert werden, woraufhin die Projektionsdaten in der Projektionsspur, also dem Strahlverlauf, die zu den Metallvoxeln beitragen, bearbeitet werden, um den Beitrag des Metalls zur Rekonstruktion zu entfernen. In Fällen starker Artefakte führt jedoch die Deformation der Metallobjekte zu einer Über- oder Untersegmentierung des Metallobjekte in der ersten Rekonstruktion, sodass die Leistungsfähigkeit solcher Algorithmen bei starken Artefakten eingeschränkt ist.

Bei MAA-Methoden wird angestrebt, die Qualität der aufgenommenen Rohdaten, also der Projektionsbilder, durch Anpassung der Aufnahmetrajektorie während der Bildaufnahme zu verbessern. Indem Projektionsbilder mit einem großen Metall-Bias vermieden werden, zeigen dreidimensionale Bilddatensätze, die aus diesen verlässlicheren Projektionsdaten rekonstruiert werden, weniger Metallartefakte und insbesondere keine zu starken Metallartefakte. Nachdem die optimierte Aufnahmetrajektorie von der Position, Form und Orientierung der Metallobjekte abhängt, werden zusätzliche Röntgenbilder, sogenannte Scoutbilder, benötigt, um eine Artefakte reduzierende, insbesondere Artefakte vermeidende, Aufnahmetrajektorie für die aufzunehmende Szene zu prädizieren. Hierbei ist es insbesondere bekannt, nach der Aufnahme der Scoutbilder eine dreidimensionale Metallmaske, die Metall enthaltende Voxel beschreibt, aus diesen zu ermitteln, um sodann Projektionsbilder aller möglichen Projektionsgeometrien zu simulieren und die beste Aufnahmetrajektorie dadurch zu bestimmen, dass die durchstrahlte Metallstrecke (Metalldurchstrahlungslänge) minimiert wird. MAA-Methoden sind beispielhaft durch US 11,790,525 B2 beschrieben.

In einem Artikel von Maximilian Rohleder et al., "An interactive task-based method for the avoidance of metal artifacts in CBCT", International Journal of Computer Assisted Radiology and Surgery (2024) 19:1399-1407, wird festgestellt, dass MAA-Methoden, die eine artefaktreduzierende Trajektorie ermitteln, aufgrund verbleibender methodischer Einschränkungen und fehlender Workflow-Integration in der klinischen Praxis noch schwer verwendbar sind. Daher wird vorgeschlagen, die räumliche Verteilung und die kalibrierten Stärken erwarteter Artefakte für eine gegebene gekippte kreisförmige Aufnahmetrajektorie zu detektieren und zu visualisieren, sodass ein Benutzer interaktiv eine optimale Aufnahmetrajektorie auswählen kann.

Ein wesentliches Problem bestehender MAA-Methoden, die eine artefaktreduzierende Aufnahmetrajektorie auf der Basis einer Metallmaske vorschlagen, ist, dass die Aufnahmetrajektorie für alle Metallvoxel des Aufnahmebereichs angepasst wird, was insbesondere dazu führen kann, dass irrelevante Objekte einen Einfluss auf die Wahl der Aufnahmetrajektorie ausüben oder sogar fälschlich im Gegensatz zu klinisch relevanten Metallobjekten, wie beispielsweise Implantaten, priorisiert werden, sodass suboptimale Aufnahmetrajektorien resultieren können. Diesbezüglich wird im genannten Artikel von Rohleder et al. vorgeschlagen, lokale Artefaktverteilungen zu ermitteln und Klinikern zu erlauben, interessierende Bereiche zu priorisieren. Ein weiteres bestehendes Problem ist, dass bei der Nutzung von Metallmasken bei der Ermittlung der optimierten Trajektorie häufig aufwändige Berechnungen und/oder Simulationen, insbesondere umfassend Vorwärtsprojektion und/oder Rendern hinsichtlich der komplexen dreidimensionalen Metallmaske, notwendig sind, die zu langen Berechnungszeiten führen.

Der Erfindung liegt die Aufgabe zugrunde, eine verbesserte, insbesondere einfach umsetzbare, rechnerisch effizient realisierbare und/oder verbessert auf das Bildgebungsziel abgestimmte Möglichkeit zur Reduzierung, insbesondere Vermeidung, von Metallartefakten während eines Aufnahmevorgangs anzugeben.

Zur Lösung dieser Aufgabe sind erfindungsgemäß ein computerimplementiertes Verfahren, eine Röntgeneinrichtung, ein Computerprogramm und ein elektronisch lesbarer Datenträger gemäß den nebengeordneten Patentansprüchen vorgesehen. Vorteilhafte Weiterbildungen ergeben sich aus den Unteransprüchen.

Bei einem erfindungsgemäßen Verfahren zum Betrieb einer Röntgeneinrichtung, die zur Aufnahme von zweidimensionalen Projektionsbildern in verschiedenen Projektionsgeometrien entlang einer Aufnahmetrajektorie, aus denen ein dreidimensionaler Bilddatensatz rekonstruierbar ist, eine Aufnahmeanordnung mit einem Röntgenstrahler und einem Röntgendetektor umfasst, ist erfindungsgemäß vorgesehen, dass vor der Aufnahme eines Aufnahmebereichs, der wenigstens ein artefaktrelevantes Metallobjekt umfasst, zur Ermittlung einer artefaktreduzierenden Aufnahmetrajektorie
- eine Objektinformation, die die dreidimensionale Position, Orientierung und Ausdehnung für jedes des wenigstens einen artefaktrelevanten Metallobjekts beschreibt, ermittelt wird,
- aus der Objektinformation für jedes artefaktrelevante Metallobjekt ein Objektmodell, das wenigstens eine dreidimensionale geometrische Grundform, die das artefaktrelevante Metallobjekt beschreibt, und deren Position, Größe und Orientierung im Aufnahmebereich beschreibt, ermittelt wird, und
- eine Artefaktmaßinformation, die die Stärke auftretender Metallartefakte beschreibt, für wenigstens eines des wenigstens einen artefaktrelevanten Metallobjekts und wenigstens eine Projektionsgeometrie, insbesondere wenigstens eine potentielle Aufnahmetrajektorie, unter Verwendung wenigstens eines des wenigstens einen Objektmodells ermittelt wird, wobei die Artefaktmaßinformation bei der Ermittlung der artefaktreduzierenden Aufnahmetrajektorie verwendet wird.

Dabei eignet sich das Verfahren insbesondere für die Kegelstrahl-Computertomographie (CBCT), bevorzugt mit einem C-Bogen, der eine Vielzahl von Freiheitsgraden hinsichtlich der Aufnahmetrajektorie bereitstellen kann. In einer konkreten Ausgestaltung kann beispielsweise vorgesehen sein, dass die artefaktreduzierende Aufnahmetrajektorie als eine um einen optimierbaren Kippwinkel gekippte Kreistrajektorie ermittelt wird. Bei einem C-Bogen kann dieser beispielsweise um den Kippwinkel gekippt werden, um die angepasste, artefaktreduzierende Aufnahmetrajektorie einzustellen. In vorteilhaften Weiterbildungen können jedoch auch komplexere artefaktreduzierende Aufnahmetrajektorien, die von Kreisbahnen abweichen, realisiert werden, indem die zu optimierenden Trajektorienparameter entsprechend gewählt werden. Rekonstruktionstechniken zur Ermittlung dreidimensionaler Bilddatensätze bei nicht kreisförmigen Aufnahmetrajektorien sind im Stand der Technik bereits bekannt.

Als artefaktrelevant können in ersten Ausgestaltungsvarianten alle in dem Aufnahmebereich und dem im dreidimensionalen Bilddatensatz rekonstruierten Rekonstruktionsbereich liegenden Metallobjekte, in deren Nähe sich Metallartefakte ausbilden können, verstanden werden, insbesondere alle Metallobjekte im Rekonstruktionsbereich. Bevorzugt und wie im Folgenden noch genauer dargelegt können artefaktrelevante Objekte in zweiten Ausgestaltungsvarianten jedoch auch durch eine Untermenge aller vorhandenen Metallobjekte gebildet sein. Bevorzugt sind solche Metallobjekte artefaktrelevant, die einer, insbesondere als Bildgebungsziel darzustellenden, Objektklasse angehören und/oder in einem als Bildgebungsziel möglichst artefaktfrei darzustellenden interessierenden Bereich liegen.

Bei dem hier dargestellten Ansatz zur Ermittlung einer artefaktreduzierenden Aufnahmetrajektorie auf Basis von Artefaktmaßinformationen handelt es sich terminologisch um ein MAA-Verfahren, also ein Verfahren der Metallartefaktvermeidung, trotzdem im Allgemeinen noch Restartefakte verbleiben können und eine vollständige Vermeidung üblicherweise nicht möglich ist. Die Artefaktvermeidung bezieht sich mithin auf Metallartefakte einer vorbestimmten Stärke und/oder Art, die insbesondere die Befundung des entstehenden Bilddatensatzes stark erschweren und/oder unmöglich machen, mithin dem Bildgebungsziel entgegenstehen. Nachdem im Allgemeinen keine vollständige Vermeidung von Artefakten möglich ist, wurde die Bezeichnung "artefaktreduzierende Aufnahmetrajektorie" gewählt. Die Artefaktreduzierung (und insbesondere Vermeidung) bezieht sich hierbei auf wenigstens eines des wenigstens einen artefaktrelevanten Metallobjekts, worauf im Folgenden noch näher eingegangen wird.

Erfindungsgemäß wird vorgeschlagen, mittels der Auswertungsfunktion artefaktrelevante Metallobjekte gezielt zu detektieren und Eigenschaften der Metallobjekte zu bestimmen, um anstatt einer abstrakten Metallmaske mit Objektmodellen arbeiten zu können, die auf einer einfachen geometrischen Grundform basieren. Mithin werden zunächst artefaktrelevante Metallobjekte detektiert und wenigstens eine Eigenschaft des jeweiligen Metallobjekts ermittelt. Dies wird durch die Objektinformation beschrieben. Jene ist geeignet, die wenigstens eine geometrische Grundform, die für das artefaktrelevante Metallobjekt verwendet werden soll, zu parametrieren, sodass sich eine klare Position, Orientierung und Ausdehnung der geometrischen Grundform als Objektmodell ergibt. Aus diesem wenigstens einen Objektmodell kann nun abgeschätzt werden, wie stark wenigstens eine Projektionsgeometrie zu Metallartefakten beiträgt bzw. bevorzugt für eine vollständige Aufnahmetrajektorie, wie stark die entstehenden Metallartefakte wohl sein werden. Das bedeutet, es wird eine Artefaktmaßinformation ermittelt, die die vorausberechnete Artefaktstärke bei der Rekonstruktion eines dreidimensionalen Bilddatensatzes entstehender Metallartefakte beschreibt. Diese kann auf verschiedene Weise gewinnbringend genutzt werden, um eine zu verwendende, artefaktreduzierende Aufnahmetrajektorie zu bestimmen, wobei diese Varianten grundsätzlich für Metallmasken bereits beschrieben wurden und auch kumulativ genutzt werden können.

Zur konkreten Ermittlung der Objektinformation existieren mehrere Möglichkeiten. So kann zum einen vorgesehen sein, dass die Objektinformation wenigstens teilweise aus Navigationsdaten wenigstens eines Navigationssystems zur Unterstützung bei einem vorangehenden Eingriff zum Positionieren der artefaktrelevanten Metallobjekte ermittelt wird. Dies ist insbesondere dann zweckmäßig, wenn die Röntgeneinrichtung, insbesondere als C-Bogen-Röntgeneinrichtung, Teil eines Eingriffsarbeitsplatzes zur Durchführung von Eingriffen, insbesondere medizinischen Eingriffen, an einem Untersuchungsobjekt, insbesondere einem Patienten, ist. Der Eingriffsarbeitsplatz kann dann auch ein Navigationssystem aufweisen, das die Position bestimmter eingriffsrelevanter Objekte, insbesondere auch der artefaktrelevanten Metallobjekte und/oder zur Positionierung der artefaktrelevanten Metallobjekte genutzter Instrumente und/oder Hilfsmittel nachverfolgt. Derartige Navigationssysteme, die auch als Nachverfolgungssysteme, Tracking-Systeme und/oder Positionsbestimmungssysteme bekannt sind, wurden im Stand der Technik bereits beschrieben. Ihre Navigationsdaten können, insbesondere bei der oben beschriebenen Nachverfolgung (Tracking) der artefaktrelevanten Metallobjekte und/oder zur Positionierung der artefaktrelevanten Metallobjekte genutzter Instrumente und/oder Hilfsmittel, bereits die Position und/oder Orientierung von artefaktrelevanten Metallobjekten in einem Koordinatensystem des Navigationssystems, das mit dem der Röntgeneinrichtung registriert ist, beschreiben. Bereits in diesem Zusammenhang kann es zweckmäßig sein, wenn wenigstens eine Vorabinformation, die die Form und Ausdehnung des Metallobjekts beschreibt, zusätzlich verwendet wird, insbesondere hinsichtlich der Ausdehnung. Somit ist in vorteilhaften Ausgestaltungen eine Ermittlung der Objektinformation insbesondere bereits ohne die Aufnahme zweidimensionaler Scoutbilder mit der Röntgeneinrichtung möglich.

Zusätzlich oder alternativ kann vorgesehen sein, dass wenigstens ein zweidimensionales Scoutbild des Aufnahmebereichs aufgenommen wird und das wenigstens eine Scoutbild mittels wenigstens einer Auswertungsfunktion zur Ermittlung der Objektinformation ausgewertet wird, die zumindest die Position, Orientierung und Ausdehnung für jedes des wenigstens einen artefaktrelevanten Metallobjekts in einem jeweiligen des wenigstens einen Scoutbilds beschreibt. Häufig werden derartige Scoutbilder auch für andere Zwecke eingesetzt, so dass deren Aufnahme oft keinen tatsächlichen Mehraufwand mit sich bringt. Auf der anderen Seite enthalten sie, ggf. zusätzlich zu Navigationsdaten, oder auch unabhängig von diesen, wertvolle Hinweise auf die Position, Orientierung und ggf. Ausdehnung der artefaktrelevanten Metallobjekte, die durch eine, bevorzugt trainierte, Auswertungsfunktion ermittelt werden können, die auf das wenigstens eine Scoutbild angewendet wird.

Hierbei kann es zweckmäßig sein, wenn wenigstens zwei zweidimensionale Scoutbilder (1) des Aufnahmebereichs in unterschiedlichen, insbesondere zueinander senkrechten, Projektionsgeometrien aufgenommen und ausgewertet werden, wobei die dreidimensionale Position, Orientierung und Ausdehnung für jedes des wenigstens einen artefaktrelevanten Metallobjekts wenigstens teilweise aus den scoutbildbezogenen Objektinformationen und den Projektionsgeometrien der Scoutbilder ermittelt werden. Beispielsweise können Referenzpunkte, die durch die Auswertungsfunktion in mehreren zweidimensionalen Scoutbildern ermittelt wurden, mittels Triangulation unter Kenntnis der Projektionsgeometrien auch dreidimensional verortet werden. Es sind jedoch auch Ausführungsbeispiele denkbar, in denen ein einziges zweidimensionales Scoutbild ausreichend ist, wenn wenigstens eine Vorabinformation, die die Form und Ausdehnung des Metallobjekts beschreibt, zur Ermittlung der dreidimensionalen Position, Orientierung und Ausdehnung für jedes des wenigstens einen artefaktrelevanten Metallobjekts verwendet wird. Häufig liegen genauere Vorabinformationen zu artefaktrelevanten Metallobjekten, die sich im Aufnahmebereich befinden, vor. Beschreiben diese die Form und die Ausdehnung hinreichend genau, kann aus der Abbildung in einem einzigen Scoutbild in vielen Fällen schon darauf geschlossen werden, wo im Raum sich das artefaktrelevante Metallobjekt befindet und wie es orientiert ist. Derartige Vorabinformation ist auch bei mehreren Scoutbildern zur genaueren Ermittlung der Objektinformation und auch bei Navigationsdaten zweckmäßig.

Es kann vorgesehen sein, dass die artefaktreduzierende Aufnahmetrajektorie und/oder wenigstens eine Vorschlagsmenge optimierter Aufnahmetrajektorien, die einem Benutzer zur Auswahl angeboten wird, in einem Optimierungsvorgang eine Zielfunktion minimierend ermittelt wird, wobei die Zielfunktion wenigstens einen aus der Artefaktmaßinformation ermittelten Artefaktmaßterm umfasst. Das bedeutet, der Ermittlung der artefaktreduzierenden Aufnahmetrajektorie kann zentral ein automatisch auf der Steuereinrichtung der Röntgeneinrichtung ablaufender Optimierungsvorgang zugrunde liegen. Dabei kann eine einzige optimierte Aufnahmetrajektorie als artefaktreduzierte, zu verwendende Aufnahmetrajektorie ermittelt werden, denkbar ist es aber auch, mehrere optimierte Aufnahmetrajektorien, beispielsweise eine Anzahl der besten Ergebnisse, auszugeben und die letztendliche Auswahl dem Benutzer zu überlassen.

Ferner kann bei der Verwendung der Artefaktmaßinformation mit besonderem Vorteil vorgesehen sein, dass die Artefaktmaßinformation metallobjektspezifisch ermittelt wird und zur objektspezifischen Visualisierung einer erwarteten Artefaktstärke pro artefaktrelevantem Metallobjekt in einer Darstellung, die der benutzerseitigen Auswahl einer zu verwendenden artefaktreduzierenden Aufnahmetrajektorie und/oder der benutzerseitigen Auswahl wenigstens eines des wenigstens einen artefaktrelevanten Metallobjekt dient, verwendet wird. Wird mithin dem Benutzer eine nach Metallobjekten aufgelöste Artefaktmaßinformation bereitgestellt, wie stark bei welcher Aufnahmetrajektorie welches artefaktrelevante Metallobjekt zu Metallartefakten beiträgt, beispielsweise für die mehreren optimierten Aufnahmetrajektorien des Optimierungsvorgangs, kann dieser eine fundierte Entscheidung für eine Aufnahmetrajektorie bzw. gegebenenfalls einen Aufnahmetrajektorienabschnitt und/oder zu verwendende Projektionsgeometrien treffen, die sich an dem Bildgebungsziel orientieren kann. Auch ist es denkbar, durch Auswahl bestimmter artefaktrelevanter Metallobjekte die Ermittlung der artefaktreduzierenden Aufnahmetrajektorie, beispielsweise den Optimierungsvorgang, auf diese zu fokussieren, um beispielsweise optimierte Bildqualität für den Bereich um diese ausgewählten artefaktrelevanten Metallobjekte zu erreichen. Beispielsweise kann also vorgesehen sein, dass bei benutzerseitiger Auswahl eines des wenigstens einen artefaktrelevanten Metallobjekts der Optimierungsvorgang bezogen auf das ausgewählte artefaktrelevante Metallobjekt durchgeführt wird. Dem liegt der Gedanke zugrunde, dass sich Metallartefakte insbesondere um das diese auslösende Metallobjekt herum ausbilden. Soll ein Metallobjekt bzw. die Anatomie um das Metallobjekt besonders deutlich zu sehen sein, bietet es sich mithin an, dieses artefaktrelevante Metallobjekt auszuwählen und somit zu priorisieren bzw. allein zu betrachten und bezüglich dieses interessierenden artefaktrelevanten Metallobjekts eine Optimierung zu erreichen bzw. eine artefaktreduzierende Aufnahmetrajektorie zu wählen, die für dieses die Bildqualität optimiert, indem Metallartefakte möglichst weitgehend vermieden werden. Die Anzeige der Artefaktmaßinformation kann beispielsweise durch Einfärbung der jeweiligen artefaktrelevanten Metallobjekte in einer abstrahierten und/oder auf einem Scoutbild basierenden Darstellung erfolgen.

Allgemein ist anzumerken, dass in dem Optimierungsvorgang, insbesondere als Term der Zielfunktion und/oder als wenigstens eine Randbedingung, die Auswertbarkeit eines resultierenden dreidimensionalen Bilddatensatzes hinsichtlich eines Bildgebungsziels und/oder die technische Durchführbarkeit der Aufnahmetrajektorie mit der Röntgeneinrichtung berücksichtigt werden kann. Allgemein gesprochen können selbstverständlich verschiedene weitere Optimierungsziele mit eingebracht werden, die auch für einen Untersuchungsvorgang spezifisch sein können, beispielsweise die Anordnung von verschiedenen Komponenten um einen aufzunehmenden Patienten herum. Insbesondere richten sich derartige weitere Terme der Zielfunktion und/oder Randbedingungen auf die grundsätzliche technische Durchführbarkeit der entstehenden artefaktreduzierten Aufnahmetrajektorie und/oder die bestmögliche Bildqualität im Hinblick auf das Bildgebungsziel. Es kann jedoch auch eine Optimierung im Hinblick auf den Komfort, beispielsweise im Umfeld eines medizinischen Eingriffs, erfolgen, indem beispielsweise eine Räumung von Bereichen von Komponenten und Personen vermieden wird.

Allgemein gesprochen wird erfindungsgemäß also, abweichend von existierenden Ansätzen, vorgeschlagen, die Metallobjekte in einer Szene explizit zu detektieren und zu parametrieren, anstatt metallische Voxel als eine dreidimensionale Metallmaske ohne Kenntnis individueller Metallobjekte abzuschätzen. Der Ansatz kann also als parametrische MAA (P-MAA) bezeichnet werden. Die Modellierung von artefaktrelevanten Metallobjekten über geometrische Grundformen erlaubt eine deutliche Erniedrigung der Berechnungszeiten für die Artefaktmaßinformation, wie Untersuchungen gezeigt haben. Dies gilt insbesondere auch, wenn Bilder, beispielsweise für Durchstrahlungslängen, gerendert werden, da auch dann die Berechnungszeiten beispielsweise um das vier- bis sechsfache reduziert werden können. Für noch im Detail zu diskutierende analytische Ansätze kann sogar eine deutlich höhere Reduzierung des Berechnungsaufwands erreicht werden, beispielsweise um das zwanzig- bis vierzigfache. Anders gesprochen erlaubt es die vorliegende Erfindung, den bislang äußerst zeit- und ressourcenaufwendigen MAA-Prozess deutlich zu beschleunigen und den Aufwand deutlich zu reduzieren. Wurden bislang Projektionsbilder der dreidimensionalen Metallmaske geändert, konnte dies selbst bei Umsetzung auf einer GPU unter Verwendung von Raytracing mehrere Minuten in Anspruch nehmen. Im hier beschriebenen Verfahren werden artefaktrelevante Metallobjekte jedoch über geometrische Grundformen modelliert, sodass auf kompakte Weise die Position, Orientierung und Ausdehnung jedes artefaktrelevanten Metallobjekts in dem Objektmodell abgebildet werden kann. Dies erlaubt eine deutliche Beschleunigung der Ermittlung der Artefaktmaßinformation, welche dennoch in hinreichend guter Qualität ermittelt werden kann, um automatisiert und/oder benutzergestützt artefaktreduzierende Aufnahmetrajektorien ermitteln zu können.

Zur Ermittlung der Artefaktmaßinformation kann zweckmäßigerweise eine Metalldurchstrahlungslänge für wenigstens eines des wenigstens einen artefaktrelevanten Metallobjekts ermittelt werden. Im Stand der Technik wurde bereits eine Mehrzahl von Prädiktoren für die Stärke von Metallartefakten vorgeschlagen, die üblicherweise allesamt auf Metalldurchstrahlungslängen basieren. Diese Methoden zur Ermittlung von Artefaktmaßinformationen, die auch im Rahmen der vorliegenden Erfindung eingesetzt werden können, umfassen beispielsweise die spektrale Verschiebungsmethode ("spectral shift"), die Streustrahlung-zu-Gesamt-Verhältnis-Methode ("scatter-to-total ratio") und die totale Schwächung ("total attenuation"). Konkret kann vorgesehen sein, dass die Metalldurchstrahlungslänge durch Rendern eines Durchstrahlungslängenbildes für die wenigstens eine Projektionsgeometrie für wenigstens eines des wenigstens einen Objektmodells erfolgt, wie bereits dargelegt wurde. Bereits bei einem solchen Ansatz der Bildsynthese lassen sich deutliche Reduzierungen des Berechnungsaufwands, insbesondere der Berechnungszeiten, erreichen. Mit besonderem Vorteil kann jedoch vorgesehen sein, dass die Berechnung der Metalldurchstrahlungslänge unter Verwendung wenigstens eines analytischen Zusammenhangs aufgrund einer Eigenschaft der geometrischen Grundform erfolgt. Bei der Verwendung eines analytischen Zusammenhangs ist besonders bevorzugt keine Ermittlung eines Durchstrahlungslängenbildes, keine Berechnung mehrerer Röntgenstrahlverläufe und keine, insbesondere aufwendige, Simulation vorgesehen oder notwendig, was eine deutliche Reduzierung des Berechnungsaufwands erlaubt und MAA-Methoden für die Nutzung in der klinischen Praxis nochmals verbessert erschließt. Darüber hinaus kann auf die Nutzung eines Grafikprozessors (GPU) verzichtet werden und allein mit üblichen Zentralprozessoren (CPUs) gearbeitet werden.

Zweckmäßigerweise wird der sogenannte "total attenuation"-Ansatz gewählt, da es sich um einen einfachen und effizienten Prädiktor für Metallartefakte handelt, insbesondere solche, die durch Photonenmangel ("photon starvation") ausgelöst werden, welcher bei medizinischen Eingriffen zum Einsetzen metallischer Implantate, beispielsweise von Pedikelschrauben, kritisch ist. Im konkreten Beispiel der Pedikelschrauben ist beispielsweise das Phänomen des Schraubenschaft-Bloomings bekannt, bei dem die Pedikelschrauben größer als ihre tatsächliche Größe erscheinen, was die Überprüfung der Platzierung im Pedikelkanal erschweren kann. Bei der "total attenuation"-Methode wird der Grad von Metallartefakten durch Beurteilung des maximalen Überschneidens von Röntgenstrahlen mit Metallobjekten beurteilt.

Um "total attenuation" zu verwenden, kann mithin vorgesehen sein, dass eine maximal durchstrahlte Metallstrecke, mithin die maximale Metalldurchstrahlungslänge, durch das wenigstens eine Objektmodell ermittelt wird. Dann lässt sich für viele geometrische Grundformen ein analytischer Zusammenhang herleiten, der es erlaubt, ohne Rendern unmittelbar für eine Durchstrahlungsrichtung, also Projektionsgeometrie, anzugeben, wie hoch die maximale Metalldurchstrahlungslänge ist. Jedoch lassen sich analytische Zusammenhänge auch für andere Metalldurchstrahlungslängen ermitteln. Insbesondere ist die Grundform derart gewählt, dass sich die längste Durchstrahlungsstrecke durch das Objektmodell aus beliebigen Projektionsrichtungen relativ zu der Grundform unmittelbar mathematisch in dem analytischen Zusammenhang ergibt. Auf diese Weise ist die Zahl der benötigten Berechnungen lediglich proportional zur Zahl der geometrischen Grundformen, sodass die Berechnungszeiten und der Berechnungsaufwand deutlich reduziert werden.

Bei einem Optimierungsvorgang kann insbesondere vorgesehen sein, dass der Artefaktmaßterm ein Durchstrahlungsterm ist, der die maximal durchstrahlte Metallstrecke durch das wenigstens eine Objektmodell für die Projektionsbilder der jeweiligen Aufnahmetrajektorie beschreibt.

Es sei angemerkt, dass auch bei einer analytischen Betrachtung von Durchstrahlungslängen durch einzelne Objektmodelle eine Berücksichtigung von mehreren potentiell entlang eines Röntgenstrahls durchstrahlten artefaktrelevanten Metallobjekten aufwandsarm erfolgen kann, beispielsweise, indem überprüft wird, ob die Objektmodelle in einer Linie liegen, und/oder indem beispielsweise überprüft wird, ob eine Hauptachse einer Grundform eine andere Grundform schneidet, welcher Anteil dann in dieser Richtung hinzuaddiert werden kann. Für viele Anwendungen kann ein Überlapp entlang Röntgenstrahlen jedoch auch vernachlässigt werden. Beispielsweise bei Pedikelschrauben liegen die Längsachsen üblicherweise hinreichend weit voneinander entfernt bzw. hinreichend weit querversetzt. Ein Überlapp hinsichtlich einer Querachse trägt bei typischen Schraubenausdehnungen weniger zum Photonenmangel bei, da die Durchmesser deutlich kleiner als die Längen sind.

Mit besonderem Vorteil kann vorgesehen sein, dass als geometrische Grundform ein Ellipsoid verwendet wird. Ellipsoiden haben sich nicht nur hinsichtlich eines potentiellen Renderns, beispielsweise von Durchstrahlungslängenbildern, als günstig in den Berechnungen erwiesen, sondern auch im Hinblick auf die Verwendung eines analytischen Zusammenhangs. So kann beispielsweise vorgesehen sein, dass die maximale Metalldurchstrahlungslänge für ein Objektmodell durch den geometrischen Schwerpunkt der Grundform, hier des Ellipsoids, des jeweiligen Objektmodells verlaufend berechnet wird. Mithin wird die Einsicht genutzt, dass die maximale Projektion eines Ellipsoiden immer durch seinen geometrischen Schwerpunkt (Zentroid) verläuft, was eine effiziente Berechnung der Artefaktmaßinformation ohne die Notwendigkeit eines Renderns erlaubt. Insbesondere ist eine äußerst drastische Reduzierung der Berechnungszeiten, beispielsweise um den Faktor 33, möglich.

Denkbar sind alternativ und/oder zusätzlich zu einem Ellipsoid auch andere geometrische Grundformen, beispielsweise Quader (beispielsweise für plattenartige Implantate), Zylinder und dergleichen. Auch hier lassen sich, wie gezeigt werden kann, analytische Zusammenhänge ermitteln. Ellipsoiden als Grundformen eignen sich insbesondere für längliche artefaktrelevante Metallobjekte wie Schrauben, Kirschnerdrähte (K-Drähte) und dergleichen. Auch allgemein kann zweckmäßig vorgesehen sein, dass wenigstens eines des wenigstens einen artefaktrelevanten Metallobjekts ein Schraubenimplantat, insbesondere eine Pedikelschraube, ist.

Es sind auch Fälle denkbar, in denen einzelne geometrische Grundformen weniger geeignet sind, artefaktrelevante Metallobjekte abzubilden. In einem solchen Fall ist es in Weiterbildung der vorliegenden Erfindung denkbar, dass wenigstens eines des wenigstens einen artefaktrelevanten Metallobjekts in dem Objektmodell durch mehrere überlagerte Grundformen modelliert wird. Auf diese Weise können auch komplexere Formen von Metallobjekten modelliert werden. Insbesondere werden wenige Grundformen, beispielsweise wenige Ellipsoiden, zur Nachbildung der komplexeren Form verwendet.

Wie bereits erwähnt wurde, kann es sich bei der Auswertungsfunktion, die auf das wenigstens eine Scoutbild angewendet wird, insbesondere um eine trainierte Auswertungsfunktion handeln.

Im Allgemeinen bildet eine trainierte Funktion kognitive Funktionen ab, die Menschen mit anderen menschlichen Gehirnen assoziieren. Durch Training basierend auf Trainingsdaten (Maschinenlernen) ist die trainierte Funktion in der Lage, sich an neue Umstände anzupassen und Muster zu detektieren und zu extrapolieren. Ein anderer Ausdruck für "trainierte Funktion" ist "trainiertes Maschinenlernmodell".

Allgemein gesagt können Parameter einer trainierten Funktion durch Training angepasst werden. Insbesondere können überwachtes Lernen, halbüberwachtes Lernen, nicht überwachtes Lernen, Reinforcement Learning und/oder aktives Lernen verwendet werden. Darüber hinaus kann auch Repräsentationslernen (auch als "feature learning" bekannt) eingesetzt werden. Die Parameter der trainierten Funktion können insbesondere iterativ durch mehrere Trainingsschritte angepasst werden. Insbesondere kann bei dem Training eine bestimmte Kostenfunktion minimiert werden. Beispielsweise kann beim Training eines neuronalen Netzes der Backpropagation-Algorithmus eingesetzt werden.

Eine trainierte Funktion kann beispielsweise ein neuronales Netz, eine Support Vector Machine (SVM), einen Entscheidungsbaum und/oder ein Bayes-Netzwerk umfassen und/oder die trainierte Funktion kann auf k-means-Clustering, Q-Learning, genetischen Algorithmen und/oder Zuordnungsregeln basieren. Insbesondere kann ein neuronales Netzwerk ein tiefes neuronales Netzwerk, ein Convolutional Neural Network (CNN) oder ein tiefes CNN sein. Darüber hinaus kann das neuronale Netzwerk ein Adversarial Network, ein tiefes Adversarial Network und/oder ein Generative Adversarial Network (GAN) sein.

Ein Convolutional Neural Network (CNN) ist ein neuronales Netz, dass eine Faltungsoperation anstatt allgemeiner Matrixmultiplikation in wenigstens einer seiner Schichten, der sogenannten Faltungsschicht, verwendet. Insbesondere kann eine Faltungsschicht ein Skalarprodukt eines oder mehrerer Faltungskerne mit den eingehenden Daten/Bildern der Faltungsschicht durchführen, wobei die Einträge des einen oder der mehreren Faltungskerne die Parameter oder Gewichte sind, die durch Training angepasst werden. Insbesondere können das innere Frobenius-Produkt und die ReLu-Aktivierungsfunktion verwendet werden. Ein CNN kann zusätzliche Schichten, beispielsweise Pooling-Schichten, vollständig verbundene Schichten und Normalisierungsschichten umfassen.

Durch CNN können Eingangsbilder, beispielsweise Scoutbilder, auf äußerst effiziente Weise verarbeitet werden, da eine Faltungsoperation basierend auf unterschiedlichen Kernen verschiedenste Bildmerkmale extrahieren kann, sodass durch Anpassung der Gewichte der Faltungskern die relevanten Bildmerkmale während des Trainings aufgefunden werden können. Darüber hinaus müssen basierend auf dem Sharing der Gewichte in den Faltungsschicht Kernen weniger Parameter trainiert werden, sodass ein Overfitting in der Trainingsphase vermieden wird und schnelleres Training oder eine größere Anzahl an Schichten in dem CNN erlaubt werden, sodass die Leistungsfähigkeit des Netzwerks erhöht wird.

In besonders vorteilhafter Weiterbildung kann vorgesehen sein, dass die trainierte Auswertungsfunktion ein regionbasiertes CNN (R-CNN) umfasst. In einem R-CNN werden Regionsvorschläge erzeugt, Merkmale extrahiert und Objekte klassifiziert, während ihre Bounding Boxes verfeinert werden. Damit kann auf äußerst schnelle und robuste Weise die Objektinformation ermittelt werden. Beispielsweise kann ein Faster R-CNN verwendet werden, dass auf einem ResNet50 basiert.

Zum Training der trainierten Auswertungsfunktion können sowohl reale als auch simulierte Bildaufnahmen verwendet werden. Zur Ermittlung simulierter Trainingsdaten können beispielsweise CBCT-Bilddatensätze ohne Metallobjekte herangezogen werden, in denen dann zufällig Metallobjekte verteilt werden und Projektionsbilder simuliert werden.

In einer besonders vorteilhaften Weiterbildung der vorliegenden Erfindung kann vorgesehen sein, dass
- wenigstens eine das wenigstens eine artefaktrelevante Metallobjekt, insbesondere dessen Objektklasse, beschreibende und von anderen, nicht zu berücksichtigenden Metallobjekten unterscheidende Hintergrundinformation bereitgestellt wird, und
- bei der Anwendung einer Auswertungsfunktion auf wenigstens ein Scoutbild die Auswertungsfunktion die Hintergrundinformation als Eingangsdaten berücksichtigt und/oder mehrere auf unterschiedliche Metallobjekte, insbesondere unterschiedliche Objektklassen, bezogene Auswertungsfunktionen bereitgestellt werden, von denen eine aufgrund der Hintergrundinformation zur Verwendung ausgewählt wird, und/oder
- bei der wenigstens teilweisen Ermittlung der Objektinformation aus Navigationsdaten die Hintergrundinformation zur Auswahl zu verwendender Navigationsdaten verwendet werden.

Auf diese Weise wird sichergestellt, dass sich die Objektinformation ausschließlich auf hinsichtlich der Entstehung von Metallartefakten und/oder deren Position im dreidimensionalen Bilddatensatz relevante Metallobjekte bezieht und weitere Metallobjekte, die als weniger bzw. nicht relevant angesehen werden, der Betrachtung entzogen werden können. Hierdurch werden beispielsweise kleinere Metallobjekte oder beispielsweise entfernt von dem eigentlich interessierenden Bereich befindliche Metallobjekte (beispielsweise chirurgische Instrumente und/oder Hilfsmittel) bei der Ermittlung der artefaktreduzierenden Aufnahmetrajektorie nicht mehr berücksichtigt, so dass insbesondere optimale Bildqualität dort erreicht werden kann, wo sie benötigt wird. Zudem können in vorbekannten Verfahren, die beispielsweise ohne Betrachtung der Objekte als solche jegliches Metall erfassende, voxelweise Metallmasken ermitteln, auftretende Probleme, wie beispielsweise Einflüsse eigentlich irrelevanter kleiner Objekte und die Priorisierung falscher Metallobjekte, vermieden werden. Die Auswertungsfunktion detektiert also gezielt Metallobjekte und ermittelt nur für jene Metallobjekte, die den zuvor ausgewählten, interessierenden, artefaktrelevanten Metallobjekten entsprechen, Objektinformationen. Entsprechend beziehen sich die verwendeten Navigationsdaten ebenso auf diese Metallobjekte. Mithin bezieht sich dann auch die Artefaktmaßinformation nur auf die artefaktrelevanten Metallobjekte. Insbesondere können die durch die Hintergrundinformation beschriebenen artefaktrelevanten Metallobjekte solche interessierenden Metallobjekte sein, deren Abbildung Ziel der Bildgebung ist, also beispielsweise Implantate, deren Sitz zu prüfen ist. Anders formuliert passen Vorgehensweisen des Standes der Technik, die mit binären Metallmasken arbeiten, die Aufnahmetrajektorie unter Berücksichtigung aller Metallvoxel einer Szene an, die irrelevante Metallobjekte neben den relevanten Objekten, also den interessierenden artefaktrelevanten Metallobjekten, umfassen können, während vorliegend lediglich die interessierenden artefaktrelevanten Metallobjekte, die durch die Hintergrundinformation beschrieben werden, modelliert werden und die anderen Metallobjekte/-voxel verworfen werden.

In konkreten Ausführungsbeispielen ist es sowohl denkbar, eine Auswertungsfunktion zu verwenden, die für mehrere Objektklassen, insbesondere diese bestimmend, trainiert ist, als auch für unterschiedliche Objektklassen trainierte Auswertungsfunktionen bereitzustellen, um dann benötigte Auswertungsfunktionen auszuwählen.

Vor einem Bildgebungsvorgang kann beispielsweise, insbesondere automatisch, ermittelt werden, welche interessierenden artefaktrelevanten Metallobjekte im Aufnahmebereich erwartet werden, und eine entsprechende Hintergrundinformation kann an die Auswertungsfunktion als Eingangsdaten weitergegeben bzw. zur Auswahl wenigstens einer geeigneten Auswertungsfunktion und/oder geeigneter Navigationsdaten verwendet werden. Beispielsweise kann das Bildgebungsziel, aus welchem die artefaktrelevanten Metallobjekte bestimmt werden können, aus Informationen automatisch hergeleitet werden, die beispielsweise in einer Patientenregistrierung erfasst wurden, in einer elektronischen Patientenakte vorliegen und/oder aus einem Krankenhausinformationssystem/Radiologieinformationssystem (HIS/RIS) und/oder einem Workflowmanagementsystem abgerufen werden können. Soll in einem Beispiel die Lage von Pedikelschrauben geprüft werden, stellen die Pedikelschrauben die artefaktrelevanten Metallobjekte dar.

Es sei angemerkt, dass bei objektaufgelöster Artefaktmaßinformation in der entsprechenden Darstellung auch eine weitere Beschränkung der artefaktrelevanten Metallobjekte, wie bereits beschrieben, aufgrund einer Benutzereingabe erfolgen kann, beispielsweise nur ausgewählte artefaktrelevante Metallobjekte bei einem Optimierungsvorgang mit einbezogen werden.

In konkreter Ausgestaltung der vorliegenden Erfindung kann vorgesehen sein, dass, insbesondere durch die Auswertungsfunktion, als Objektinformation für jedes artefaktrelevante Metallobjekt eine Objektklasse und/oder wenigstens zwei ausgezeichnete Referenzpunkte des Metallobjekts und/oder wenigstens eine Bounding Box um das Metallobjekt ermittelt wird. Insbesondere können die Referenzpunkte und/oder die Bounding Box so gewählt sein, dass die geometrische Grundform möglichst einfach parametriert werden kann. In einem konkreten Beispiel kann vorgesehen sein, dass die Referenzpunkte bei einem länglichen artefaktrelevanten Metallobjekt wenigstens zwei die Länge in der Richtung größter Ausdehnung beschreibende Schlüsselpunkte sind. Für eine Pedikelschraube kann beispielsweise ein Schlüsselpunkt an der Schraubenspitze (Ende des Schraubenschafts) und ein Schlüsselpunkt am Schraubenkopf entlang der Längsrichtung vorgesehen sein. Wird die Pedikelschraube als Ellipsoid modelliert, kann dessen Hauptachse in die durch die Schlüsselpunkte beschriebenen Kopf-zu-Spitze-Richtung ausgerichtet und gemäß dem Kopf-zu-Spitze-Abstand skaliert werden. Die zweiten und dritten Achsen des Ellipsoids können gemäß einer Vorgabe, die übliche Durchmesser von Pedikelschrauben beschreiben, beispielsweise 5 mm, dimensioniert werden.

Zweckmäßigerweise kann der Objektklasse jeweils eine zu verwendende Grundform zugeordnet sein. Für unterschiedliche Objektklassen kann es sinnvoll sein, unterschiedliche Grundformen zu verwenden, wie bereits angedeutet wurde. Besonders einfach ist dabei eine Zuordnung von Grundformen zu Objektklassen, die durch die, insbesondere trainierte, Auswertungsfunktion ohnehin bestimmt werden. So kann auf einfache Weise eine korrekte, auf die Objektklasse angepasste Modellierung erfolgen.

Neben dem Verfahren betrifft die vorliegende Erfindung auch eine Röntgeneinrichtung, aufweisend eine Steuereinrichtung und eine Aufnahmeanordnung zur Aufnahme von zweidimensionalen Projektionsbildern in verschiedenen Projektionsgeometrien entlang einer Aufnahmetrajektorie, aus denen ein dreidimensionaler Bilddatensatz rekonstruierbar ist, wobei die Aufnahmeanordnung einen Röntgenstrahler und einen Röntgendetektor umfasst, wobei die Steuereinrichtung zur Durchführung eines erfindungsgemäßen Verfahrens eingerichtet ist. Sämtliche Ausführungen bezüglich des erfindungsgemäßen Verfahrens lassen sich analog auf die erfindungsgemäße Röntgeneinrichtung übertragen, mit welcher mithin ebenso die bereits genannten Vorteile erhalten werden können.

Die Steuereinrichtung umfasst wenigstens einen Prozessor und wenigstens ein Speichermittel und kann durch Hardware und/oder Software gebildete Funktionseinheiten aufweisen, um Schritte des erfindungsgemäßen Verfahrens durchzuführen. Insbesondere kann die Steuereinrichtung, wie grundsätzlich bekannt, eine Aufnahmeeinheit aufweisen, um den Aufnahmebetrieb der Aufnahmeanordnung zu steuern. Die Aufnahmeeinheit kann beispielsweise eingerichtet sein, vor der Aufnahme von Projektionsbildern, aus denen ein dreidimensionaler Bilddatensatz rekonstruiert werden soll, eines Aufnahmebereichs, der wenigstens ein artefaktrelevantes Metallobjekt umfasst, wenigstens ein zweidimensionales Scoutbild des Aufnahmebereichs, insbesondere wenigstens zwei Scoutbilder in unterschiedlichen, insbesondere zueinander senkrechten, Projektionsgeometrien, aufzunehmen. Ferner kann die Aufnahmeeinheit ausgebildet sein, die Projektionsbilder entlang der im erfindungsgemäßen Verfahren ermittelten artefaktreduzierenden Aufnahmetrajektorie aufzunehmen. Die Steuereinrichtung kann ferner eine Auswerteeinheit umfassen, um die Objektinformation zu ermitteln. Hierzu kann die Auswerteeinheit eingerichtet sein, um das wenigstens eine Scoutbild mittels wenigstens einer, insbesondere trainierten, Auswertungsfunktion zur Ermittlung der Objektinformation auszuwerten, die die Position, Orientierung und Ausdehnung für jedes des wenigstens einen artefaktrelevanten Metallobjekts in den jeweiligen Scoutbildern beschreibt. Ferner kann die Auswerteeinheit ausgebildet sein, Navigationsdaten eines Navigationssystems hinsichtlich der Objektinformation auszuwerten. Die Steuereinrichtung kann weiterhin eine erste Ermittlungseinheit zur Ermittlung eines Objektmodells, das wenigstens eine dreidimensionale geometrische Grundform, die das artefaktrelevante Metallobjekt beschreibt, und deren Position, Größe und Orientierung im Aufnahmebereich beschreibt, für jedes artefaktrelevante Metallobjekt aus der Objektinformation aufweisen. Eine zweite Ermittlungseinheit kann vorgesehen sein, um eine Artefaktmaßinformation, die die Stärke auftretender Metallartefakte prädiziert, für wenigstens eines des wenigstens einen artefaktrelevanten Metallobjekts und wenigstens eine Projektionsgeometrie, insbesondere wenigstens eine potentielle Aufnahmetrajektorie, unter Verwendung wenigstens eines des wenigstens einen Objektmodells zu ermitteln. Zur Verwendung der Artefaktmaßinformation bei der Ermittlung der artefaktreduzierenden Aufnahmetrajektorie kann eine Verwendungseinheit der Steuereinrichtung eingesetzt werden. Die Verwendungseinheit kann beispielsweise eine Optimierungseinheit umfassen oder sein, die eingerichtet ist, die artefaktreduzierende Aufnahmetrajektorie in einem Optimierungsvorgang eine Zielfunktion minimierend zu ermitteln, wobei die Zielfunktion wenigstens einen aus der Artefaktmaßinformation ermittelten Artefaktmaßterm umfasst. Zusätzlich oder alternativ kann die Verwendungseinheit eine Darstellungseinheit umfassen oder sein, die zur objektspezifischen Visualisierung einer erwarteten Artefaktstärke pro artefaktrelevantem Metallobjekt in einer Darstellung, die der benutzerseitigen Auswahl einer zu verwendenden artefaktreduzierenden Aufnahmetrajektorie und/oder der benutzerseitigen Auswahl wenigstens eines des wenigstens einen artefaktrelevanten Metallobjekts dient, eingerichtet ist. Für weitere Ausgestaltungen des Verfahrens können auch weitere Funktionseinheiten vorgesehen werden, beispielsweise eine Schnittstelle zum Empfang der Hintergrundinformation und/oder eine Hintergrundinformationseinheit zu deren Ermittlung.

Die Röntgeneinrichtung kann bevorzugt einen C-Bogen umfassen, an dem sich gegenüberliegend der Röntgenstrahler und der Röntgendetektor angeordnet sind. Über entsprechende Bewegungsfreiheitsgrade des C-Bogens sowie gegebenenfalls des Röntgenstrahlers und/oder des Röntgendetektors können verschiedenste Aufnahmetrajektorien realisiert werden, von denen die hinsichtlich der Artefakte für artefaktrelevante Metallobjekte optimale Strahler-Detektor-Aufnahmetrajektorie als artefaktreduzierende Aufnahmetrajektorie ausgewählt werden kann. C-Bogen-Röntgeneinrichtungen werden häufig bei medizinischen Interventionen zu deren Überwachung und zur Kontrolle des Behandlungserfolgs eingesetzt, auch durch Aufnahme von dreidimensionalen CBCT-Bilddatensätzen. Die Röntgeneinrichtung kann Teil eines Eingriffsarbeitsplatzes sein, der neben der Röntgeneinrichtung auch ein Navigationssystem umfassen kann, wie bereits beschrieben.

Ein erfindungsgemäßes Computerprogramm ist direkt in ein Speichermittel einer Steuereinrichtung einer Röntgeneinrichtung ladbar und weist Programmmittel derart auf, dass bei Ausführen des Computerprogramms die Steuereinrichtung veranlasst wird, die Schritte eines erfindungsgemäßen Verfahrens durchzuführen. Das Computerprogramm kann auf einem elektronisch lesbaren Datenträger gemäß der vorliegenden Erfindung gespeichert sein, der mithin darauf gespeicherte Steuerinformation umfasst, die wenigstens ein erfindungsgemäßes Computerprogramm umfassen und derart ausgestaltet sind, dass bei Verwendung des Datenträgers in einer Steuereinrichtung einer Magnetresonanzeinrichtung diese zur Durchführung eines erfindungsgemäßen Verfahrens ausgebildet wird. Bei dem Datenträger kann es sich insbesondere um einen nicht-transienten Datenträger, beispielsweise eine CD-ROM, handeln.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen Ablaufplan eines Ausführungsbeispiels des erfindungsgemäßen Verfahrens,
- Fig. 2: schematisch ein Scoutbild,
- Fig. 3: schematisch ein dreidimensionales Objektmodell,
- Fig. 4: eine mögliche Darstellung zur Anzeige von Artefaktmaßinformation,
- Fig. 5: eine erfindungsgemäße Röntgeneinrichtung, und
- Fig. 6: den funktionalen Aufbau einer Steuereinrichtung der Röntgeneinrichtung.

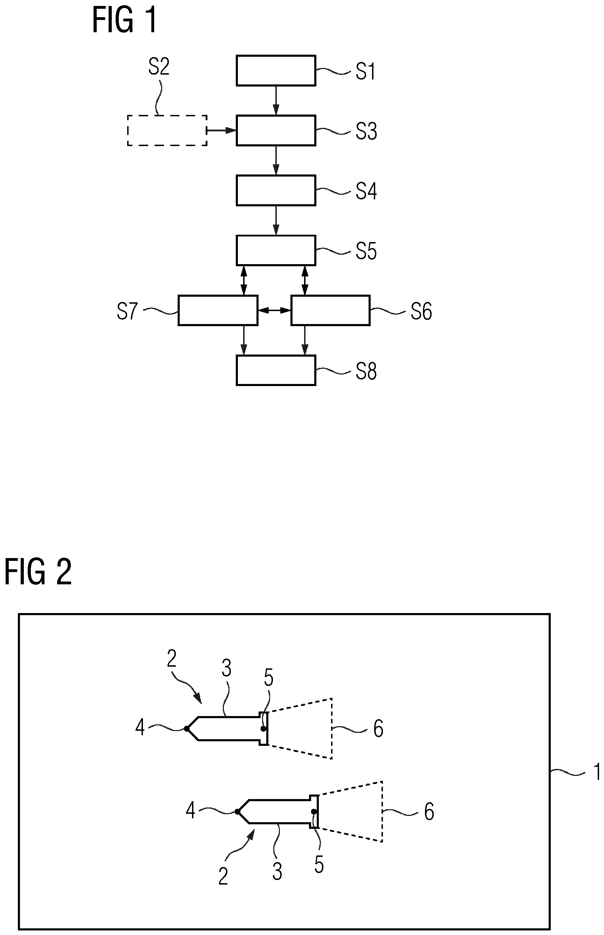

Fig. 1 zeigt einen Ablaufplan eines Ausführungsbeispiels des erfindungsgemäßen Verfahrens. Dieses dient dazu, an einer CBCT-tauglichen Röntgeneinrichtung, an der eine Vielzahl von Projektionsgeometrien eingestellt werden können, eine zu verwendende artefaktreduzierende Aufnahmetrajektorie zu ermitteln, wenn Metallobjekte in einem Aufnahmebereich befindlich sind. Entlang der Aufnahmetrajektorie sollen Projektionsbilder unter Nutzung verschiedener Projektionsgeometrien aufgenommen werden, um hieraus einen dreidimensionalen Bilddatensatz des Aufnahmebereichs zu rekonstruieren. Das konkrete Beispiel bezieht sich dabei auf die Kontrolle der korrekten Positionierung von metallischen Implantaten, wobei konkret - und rein beispielhaft - Pedikelschrauben betrachtet werden. Selbstverständlich erschließen sich dem fachkundigen Leser jedoch auch eine Vielzahl anderer Anwendungsmöglichkeiten des erfindungsgemäßen Verfahrens.

In einem Schritt S1 werden dazu zunächst in diesem Ausführungsbeispiel wenigstens zwei, insbesondere genau zwei, Scoutbilder des Aufnahmebereichs aufgenommen, wobei unterschiedliche Aufnahmegeometrien verwendet werden, deren Zentralstrahlen aufeinander senkrecht stehen. In dem Aufnahmebereich befindliche Metallobjekte werden dabei erfasst. Es sind auch Ausgestaltungen denkbar, in denen nur ein Scoutbild erfasst wird oder sogar gänzlich ohne Scoutbilder gearbeitet wird, beispielsweise stattdessen in einem ersten Schritt Navigationsdaten eines Navigationssystems empfangen werden, die Metallobjekte in dem Aufnahmebereich betreffen.

In einem optionalen Schritt S2, der Scoutbilder nutzen kann, bevorzugt aber unabhängig von diesen durchgeführt wird, wird eine Hintergrundinformation ermittelt und bereitgestellt. Die Hintergrundinformation beschreibt, welche der Gesamtheit der möglicherweise vorhandenen Metallobjekte artefaktrelevant sind, beispielsweise in Form wenigstens einer Objektklasse. Dies kann beispielsweise aus einer Benutzereingabe oder aber automatisch, beispielsweise aus einem seitens eines Informationssystems vorliegenden Bildgebungsziel, ermittelt werden. Vorliegend sind beispielsweise Pedikelschrauben die artefaktrelevanten Metallobjekte, da deren Sitz überprüft werden soll und mithin in deren Nähe ein dreidimensionaler, rekonstruierter Bilddatensatz möglichst artefaktarm sein soll.

In einem Schritt S3 wird eine, vorliegend trainierte, Auswertungsfunktion auf die Scoutbilder angewendet, um Objektinformationen zu jedem artefaktrelevanten Objekt zu ermitteln. Vorliegend wird eine trainierte Auswertungsfunktion speziell für Pedikelschrauben verwendet; es sind auch generellere Auswertungsfunktionen für mehrere Objektklassen denkbar. In Ausführungsbeispielen kann es denkbar sein, aufgrund der Hintergrundinformation wenigstens eine geeignete Auswertungsfunktion auszuwählen, wie hier geschehen.

Die trainierte Auswertungsfunktion nutzt vorliegend eine "Faster R-CNN"-Architektur und liefert als Objektinformationen für jedes artefaktrelevante Metallobjekt eine zweidimensionale Bounding Box und vorliegend als Referenzpunkte zwei Schlüsselpunkte, einen für die Spitze der Pedikelschraube, einen für den Kopf. Dies illustriert Fig. 2 als rein schematische Darstellung eines Scoutbildes 1. Dieses zeigt beispielhaft als artefaktrelevante Metallobjekte 2 zwei Pedikelschrauben 3. Als Referenzpunkte liefert die Objektinformation Schlüsselpunkte 4 an den Spitzen des Schafts und Schlüsselpunkte 5 an den Schraubenköpfen. Weitere, nicht artefaktrelevante Metallobjekte 6, umfassend sogenannte Tulpen und/oder "Türme" an den Schraubenköpfen sind angedeutet. Diese werden bei der Ermittlung einer geeigneten artefaktreduzierenden Aufnahmetrajektorie von Röntgenstrahler und Röntgendetektor nicht weiter berücksichtigt, so dass eine Optimierung auf die Pedikelschrauben 3 fokussiert.

Werden in anderen Ausführungsbeispielen nur oder zusätzlich Navigationsdaten verwendet, kann die Hintergrundinformation zur Selektion relevanter Anteile der Navigationsdaten verwendet werden und Schritt S4 deren Auswertung zur Ermittlung der Objektinformationen umfassen.

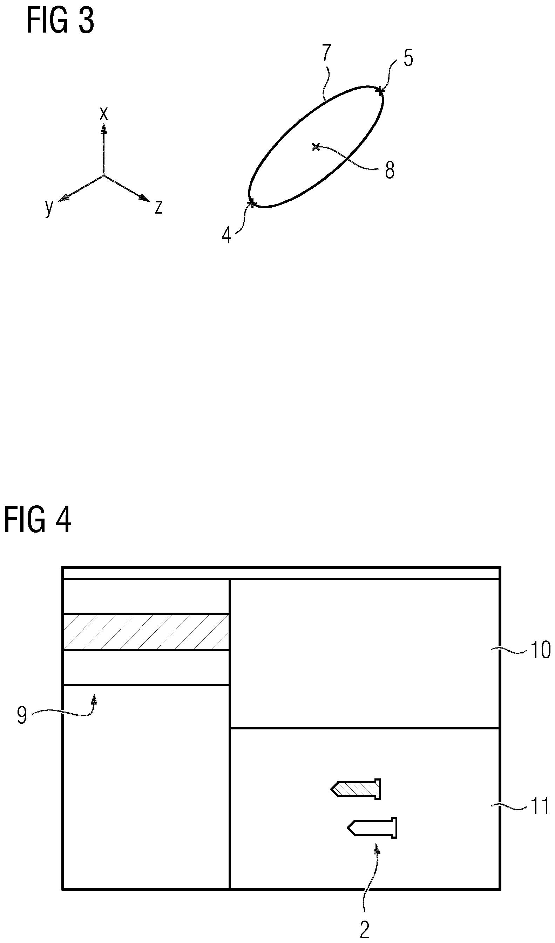

In einem Schritt S4 wird auf Basis der Objektinformationen für jedes artefaktrelevante Metallobjekt ein dreidimensionales Objektmodell auf Basis einer dreidimensionalen geometrischen Grundform (also insbesondere eines geometrischen Primitivs) ermittelt. Die Pedikelschrauben 3 als längliche artefaktrelevante Metallobjekte 2 werden vorliegend, vergleiche Fig. 3, durch Ellipsoiden 7 abgebildet, deren längste Hauptachse zwischen den Schlüsselpunkten 4 und 5 der jeweiligen Pedikelschraube verläuft, die aufgrund der mehreren Scoutbilder 1 in drei Dimensionen bestimmt werden können. Die Ausdehnung der weiteren Achsen des Ellipsoiden 7 wird gemäß üblicher Durchmesser solcher Pedikelschrauben 3 gewählt, beispielsweise als 5 mm. Dabei ist zur besseren Darstellbarkeit der Ellipsoid der Fig. 3 etwas breiter gezeigt als er in der Praxis üblicherweise ist. Gezeigt ist in Fig. 3 auch der geometrische Schwerpunkt 8 des Ellipsoiden 7.

In einem Schritt S5 werden die Objektmodelle genutzt, um eine Artefaktmaßinformation zu ermitteln, die dann verwendet wird, um die artefaktreduzierende Aufnahmetrajektorie zu ermitteln. Vorliegend wird ein "Total Attenuation"-Ansatz gewählt, in dem als Maß für den Beitrag zur Artefaktstärke die maximale Metalldurchstrahlungslänge verwendet wird. Für jede, beispielsweise im Fall eines C-Bogen durch zwei Winkel charakterisierte, Projektionsgeometrie, die entlang einer Aufnahmetrajektorie genutzt werden kann, kann anhand der Objektmodelle, insbesondere objektmodellspezifisch und somit für jedes artefaktrelevante Metallobjekt spezifisch die Artefaktmaßinformation ermittelt werden. Während es grundsätzlich denkbar ist, insbesondere bei nahe beieinander liegenden bzw. komplex angeordneten artefaktrelevanten Metallobjekten, Metalldurchstrahlungslängenbilder deutlich schneller als bei Verwendung einer dreidimensionalen Metallmaske zu rendern, erlaubt es die Verwendung von Grundformen, diese für einzelne Objektmodelle auch analytisch zu bestimmen. So lässt sich bei Ellipsoiden 7 auf einfache Weise die maximale Metalldurchstrahlungslänge analytisch bestimmen, denn sie verläuft immer durch den geometrischen Schwerpunkt 8.

Im Anwendungsfall der Pedikelschrauben 3 liegen diese räumlich meist hinreichend getrennt, so dass entlang der Schäfte eine Überlagerung nicht zu befürchten ist; quer hierzu ist sie weniger kritisch, da es bei den geringeren Durchmessern üblicherweise nicht zu einem Absinken der Photonenzahl unter einen kritischen Wert ("photon starvation") kommt. Es kann jedoch auch vorgesehen sein, rechnerisch auch bei Nutzung eines analytischen Zusammenhangs pro Objektmodell eine Überlagerung zu ermitteln und zu berücksichtigen.

Es ist im Schritt S5 möglich, eine Art "Metrikkarte" als Artefaktmaßinformation zu ermitteln, indem für denkbare/umsetzbare Projektionsgeometrien jeweils die maximalen Metalldurchstrahlungslängen ermittelt werden. Bei einem C-Bogen und durch zwei Winkel charakterisierten Projektionsgeometrien können beispielsweise in 1°-Schritten erlaubte Kombinationen abgedeckt werden. Wird eine Artefaktmaßinformation für eine Aufnahmetrajektorie benötigt, können die entsprechenden Werte aus der Metrikkarte abgerufen werden. Selbstverständlich ist es aber auch möglich, die Artefaktmaßinformation für bestimmte Aufnahmetrajektorien immer gezielt zu ermitteln.

Die Artefaktmaßinformation kann bei der Ermittlung der artefaktreduzierenden Aufnahmetrajektorie auf unterschiedliche Art und Weise konkret eingesetzt werden, wobei die zentralen Verwendungsarten, die auch zusammenwirken können, in Fig. 1 schematisch als Schritte S6, S7 angedeutet sind.

In einem Schritt S6 erfolgt ein Optimierungsvorgang unter Verwendung der Artefaktmaßinformation. Dabei wird eine Zielfunktion verwendet, die einen Artefaktmaßterm enthält, der aus der Artefaktmaßinformation abgeleitet ist und mithin auf die Minimierung der Artefaktstärke für die artefaktrelevanten Metallobjekte 2 abzielt. Die Zielfunktion kann auch weitere Terme aufweisen bzw. der Optimierungsvorgang im allgemeinen Randbedingungen nutzen, um Aspekte wie die Durchführbarkeit der Aufnahmetrajektorien und dergleichen abzubilden. Der Optimierungsvorgang kann eine einzige artefaktreduzierende Aufnahmetrajektorie, aber auch mehrere artefaktreduzierende Aufnahmetrajektorien ausgeben, welche beispielsweise verschiedenen lokalen Minima, die sich durch unterschiedliche Beiträge unterschiedlicher artefaktrelevanter Metallobjekte ergeben können, zugeordnet sein können. In einer einfach umsetzbaren und dennoch zweckmäßigen Variante kann die Optimierung auf die Ermittlung wenigstens eines optimierten Kippwinkels des C-Bogens abzielen, um den auch eine Kreisbahn als Aufnahmetrajektorie verkippt wird. Selbstverständlich kann die artefaktreduzierende Aufnahmetrajektorie jedoch auch unter Nutzung weiterer Freiheitsgrade, insbesondere abweichend von einer Kreisbahn, ermittelt werden, insbesondere für beliebige Wege und/oder durch einen weiteren Term der Zielfunktion derart gewählte Wege, dass eine robuste Rekonstruktion möglich bleibt.

Der Schritt S7 symbolisiert eine Anzeige der Artefaktmaßinformation in einer Darstellung, die zur benutzerseitigen Auswahl einer geeigneten artefaktreduzierenden Aufnahmetrajektorie dienen kann, aber auch die Auswahl bestimmter, beispielsweise besonders interessierender, artefaktrelevanter Metallobjekte 2 erlauben kann, für die dann erneut optimiert wird. Beispielsweise ist es denkbar, für durch einen Benutzer vorgegebene Kippwinkel für Kreisbahnen, was beispielsweise durch Interaktion mit der Darstellung erfolgen kann, immer die zugeordneten Artefaktmaßinformationen anzuzeigen, insbesondere aufgelöst nach artefaktrelevanten Metallobjekten. Auch kann die Darstellung dazu dienen, bei mehreren von einem Optimierungsvorgang ermittelten optimierten Aufnahmetrajektorien eine davon auszuwählen.

Fig. 4 zeigt hierzu beispielsweise eine Darstellung mit mehreren optimierten und empfohlenen, beispielsweise in einem Optimierungsvorgang ermittelten Aufnahmetrajektorien, die in einer Liste 9 auswählbar sind. In einem Teilfenster 10 werden gesamte Artefaktmaßinformationen (also für alle artefaktrelevanten Metallobjekte 2) vergleichbar für alle Aufnahmetrajektorien angezeigt, während in einem Teilfenster 11 - die nach Metallobjekten 2 aufgelöste Ermittlung ausnutzend - ein beispielsweise aus einem Scoutbild 1 abgeleitetes oder dieses umfassendes Darstellungsbild angezeigt wird, in dem die artefaktrelevanten Metallobjekte 2 gemäß ihrem Anteil an den prädizierten Metallartefaktstärken gekennzeichnet, beispielsweise eingefärbt sind. So kann ein Benutzer eine artefaktreduzierende Aufnahmetrajektorie auswählen, die bei für ihn besonders interessierenden artefaktrelevanten Metallobjekten 2, beispielsweise neu eingesetzten oder zuletzt positionierten Pedikelschrauben 3, die geringste Artefaktstärke zeigt.

In einem Schritt S8 kann dann, wenn eine artefaktreduzierende Aufnahmetrajektorie, die zu verwenden ist, ermittelt ist, die Aufnahme entlang der ermittelten artefaktreduzierten Aufnahmetrajektorie erfolgen.

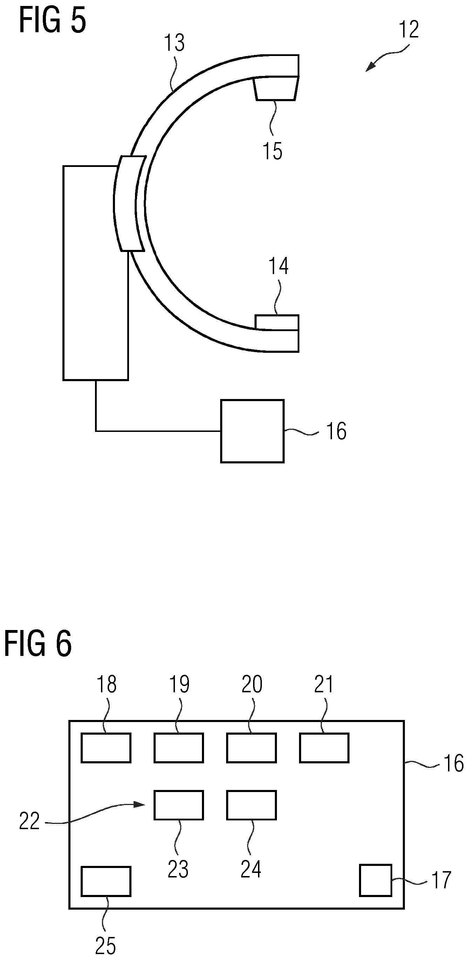

Fig. 5 zeigt eine Prinzipskizze einer erfindungsgemäßen Röntgeneinrichtung 12. Diese umfasst vorliegend einen C-Bogen 13, an dem sich gegenüberliegend ein Röntgenstrahler 14 und ein Röntgendetektor 15 angeordnet sind. Der Betrieb der Röntgeneinrichtung 12 wird mittels einer Steuereinrichtung 16 gesteuert, die auch zur Durchführung des erfindungsgemäßen Verfahrens ausgebildet ist. Insbesondere kann die Steuereinrichtung 16 Aktoren ansteuern, um verschiedene Projektionsgeometrien entlang einer, insbesondere artefaktreduzierenden, Aufnahmetrajektorie durch Bewegung des C-Bogens 13 anzusteuern.

Fig. 6 zeigt die funktionale Struktur der Steuereinrichtung 16 genauer. Die Steuereinrichtung 16 umfasst zunächst wenigstens ein Speichermittel 17, in dem beispielsweise Scoutbilder 1, Artefaktmaßinformationen, Projektionsbilder, artefaktreduzierende Aufnahmetrajektorien und dergleichen zumindest temporär abgelegt werden können. Die Steuereinrichtung 16 umfasst ferner, wie grundsätzlich bekannt, eine Aufnahmeeinheit 18, um den Aufnahmebetrieb der durch den Röntgenstrahler 14 und den Röntgendetektor 15 gebildeten Aufnahmeanordnung und die durch den C-Bogen 13 bereitgestellte Projektionsgeometrie zu steuern. Die Aufnahmeeinheit 18 ist zum einen eingerichtet, vor der Aufnahme der Projektionsbildern, aus denen der dreidimensionale Bilddatensatz rekonstruiert werden soll, die wenigstens zwei zweidimensionalen Scoutbilder 1 des Aufnahmebereichs gemäß Schritt S1 aufzunehmen. Außerdem ist die Aufnahmeeinheit 18 ausgebildet, die Projektionsbilder entlang der im erfindungsgemäßen Verfahren ermittelten artefaktreduzierenden Aufnahmetrajektorie gemäß Schritt S8 aufzunehmen.

Die Steuereinrichtung 16 umfasst vorliegend ferner eine Auswerteeinheit 19, um die Scoutbilder 1 gemäß dem Schritt S3 mittels der wenigstens einen trainierten Auswertungsfunktion zur Ermittlung der Objektinformation auszuwerten. Zudem ist eine erste Ermittlungseinheit 20 zur Ermittlung der Objektmodelle für jedes artefaktrelevante Metallobjekt 2 aus der Objektinformation gemäß Schritt S4 vorgesehen. Eine zweite Ermittlungseinheit 21 ist eingerichtet, die Artefaktmaßinformation, die die Stärke auftretender Metallartefakte beschreibt, für wenigstens eines des wenigstens einen artefaktrelevanten Metallobjekts 2 und wenigstens eine Projektionsgeometrie, insbesondere wenigstens eine potentielle Aufnahmetrajektorie, gemäß dem Schritt S5 vorauszuberechnen. Zur Verwendung der Artefaktmaßinformation bei der Ermittlung der artefaktreduzierenden Aufnahmetrajektorie sind vorliegend zwei Verwendungseinheiten 22 der Steuereinrichtung 16 gezeigt. Eine Optimierungseinheit 23 ist zur Durchführung des Optimierungsvorgangs gemäß Schritt S6 ausgebildet. Zusätzlich oder alternativ ist eine Darstellungseinheit 24 vorhanden, die zur objektspezifischen Visualisierung der erwarteten Artefaktstärke pro artefaktrelevantem Metallobjekt 2 in einer Darstellung gemäß Schritt S7 dient. Für den optionalen Schritt S2 ist zudem eine Hintergrundinformationseinheit 25 zur Ermittlung der Hintergrundinformation vorgesehen.

Die Röntgeneinrichtung 12 kann Teil eines Eingriffsarbeitsplatzes sein, der auch ein hier nicht näher dargestelltes Navigationssystem umfassen kann. Mittels des Navigationssystems können artefaktrelevante Metallobjekte und/oder zu deren Positionierung genutzte Instrumente und/oder Hilfsmittel in einem mit dem Koordinatensystem der Röntgeneinrichtung registrierten Koordinatensystem nachverfolgt werden. Entsprechende Navigationsdaten können über eine Schnittstelle der Steuereinrichtung 16 bereitgestellt werden.

Unabhängig vom grammatikalischen Geschlecht eines bestimmten Begriffes sind Personen mit männlicher, weiblicher oder anderer Geschlechteridentität mit umfasst.

## Patentansprüche

1. Computerimplementiertes Verfahren zum Betrieb einer Röntgeneinrichtung (12), die zur Aufnahme von zweidimensionalen Projektionsbildern in verschiedenen Projektionsgeometrien entlang einer Aufnahmetrajektorie, aus denen ein dreidimensionaler Bilddatensatz rekonstruierbar ist, eine Aufnahmeanordnung mit einem Röntgenstrahler (14) und einem Röntgendetektor (15) umfasst, wobei vor der Aufnahme eines Aufnahmebereichs, der wenigstens ein artefaktrelevantes Metallobjekt (2) umfasst, zur Ermittlung einer artefaktreduzierenden Aufnahmetrajektorie
- eine Objektinformation, die die dreidimensionale Position, Orientierung und Ausdehnung für jedes des wenigstens einen artefaktrelevanten Metallobjekts (2) beschreibt, ermittelt wird,
- aus der Objektinformation für jedes artefaktrelevante Metallobjekt (2) ein Objektmodell, das wenigstens eine dreidimensionale geometrische Grundform, die das artefaktrelevante Metallobjekt (2) beschreibt, und deren Position, Größe und Orientierung im Aufnahmebereich beschreibt, ermittelt wird, und
- eine Artefaktmaßinformation, die die Stärke auftretender Metallartefakte beschreibt, für wenigstens eines des wenigstens einen artefaktrelevanten Metallobjekts (2) und wenigstens eine Projektionsgeometrie unter Verwendung wenigstens eines des wenigstens einen Objektmodells ermittelt wird, wobei die Artefaktmaßinformation bei der Ermittlung der artefaktreduzierenden Aufnahmetrajektorie verwendet wird.

2. Verfahren nach Anspruch 1, **dadurch gekennzeichnet, dass**
- die Objektinformation wenigstens teilweise aus Navigationsdaten wenigstens eines Navigationssystems zur Unterstützung bei einem vorangehenden Eingriff zum Positionieren der artefaktrelevanten Metallobjekte (2) ermittelt wird und/oder
- wenigstens ein zweidimensionales Scoutbild (1) des Aufnahmebereichs aufgenommen wird und das wenigstens eine Scoutbild (1) mittels wenigstens einer Auswertungsfunktion zur Ermittlung der Objektinformation ausgewertet wird, die zumindest die Position, Orientierung und Ausdehnung für jedes des wenigstens einen artefaktrelevanten Metallobjekts (2) in einem jeweiligen des wenigstens einen Scoutbilds (2) beschreibt.

3. Verfahren nach Anspruch 2, **dadurch gekennzeichnet, dass** wenigstens zwei zweidimensionale Scoutbilder (1) des Aufnahmebereichs in unterschiedlichen Projektionsgeometrien aufgenommen und ausgewertet werden, wobei die dreidimensionale Position, Orientierung und Ausdehnung für jedes des wenigstens einen artefaktrelevanten Metallobjekts (2) wenigstens teilweise aus den scoutbildbezogenen Objektinformationen und den Projektionsgeometrien der Scoutbilder (1) ermittelt werden, und/oder dass, insbesondere bei Verwendung eines einzigen Scoutbilds (1), wenigstens eine Vorabinformation, die die Form und Ausdehnung des Metallobjekts (2) beschreibt, zur Ermittlung der dreidimensionalen Position, Orientierung und Ausdehnung für jedes des wenigstens einen artefaktrelevanten Metallobjekts (2) verwendet wird.

4. Verfahren nach einem der vorangehenden Ansprüche, **dadurch gekennzeichnet, dass** die artefaktreduzierende Aufnahmetrajektorie und/oder wenigstens eine Vorschlagsmenge optimierter Aufnahmetrajektorien, die einem Benutzer zur Auswahl angeboten wird, in einem Optimierungsvorgang eine Zielfunktion minimierend ermittelt wird, wobei die Zielfunktion wenigstens einen aus der Artefaktmaßinformation ermittelten Artefaktmaßterm umfasst.

5. Verfahren nach einem der vorangehenden Ansprüche, **dadurch gekennzeichnet, dass** die Artefaktmaßinformation nach Metallobjekten aufgelöst ermittelt wird und zur objektspezifischen Visualisierung einer erwarteten Artefaktstärke pro artefaktrelevantem Metallobjekt (2) in einer Darstellung, die der benutzerseitigen Auswahl einer zu verwendenden artefaktreduzierenden Aufnahmetrajektorie und/oder der benutzerseitigen Auswahl wenigstens eines des wenigstens einen artefaktrelevanten Metallobjekts (2) dient, verwendet wird.

6. Verfahren nach einem der vorangehenden Ansprüche, **dadurch gekennzeichnet, dass** zur Ermittlung der Artefaktmaßinformation eine Metalldurchstrahlungslänge für wenigstens eines des wenigstens einen artefaktrelevanten Metallobjekts (2) ermittelt wird.

7. Verfahren nach Anspruch 6, **dadurch gekennzeichnet, dass** die Metalldurchstrahlungslänge durch Rendern eines Durchstrahlungslängenbildes für die wenigstens eine Projektionsgeometrie für wenigstens eines des wenigstens einen Objektmodells erfolgt und/oder dass die Berechnung der Metalldurchstrahlungslänge unter Verwendung wenigstens eines analytischen Zusammenhangs aufgrund einer Eigenschaft der geometrischen Grundform erfolgt.

8. Verfahren nach einem der vorangehenden Ansprüche, **dadurch gekennzeichnet, dass** als geometrische Grundform ein Ellipsoid (7) verwendet wird.

9. Verfahren nach einem der vorangehenden Ansprüche, **dadurch gekennzeichnet, dass** wenigstens eines des wenigstens einen artefaktrelevanten Metallobjekts (2) in dem Objektmodell durch mehrere überlagerte Grundformen modelliert wird.

10. Verfahren nach einem der vorangehenden Ansprüche, **dadurch gekennzeichnet, dass**
- wenigstens eine das wenigstens eine artefaktrelevante Metallobjekt (2), insbesondere dessen Objektklasse, beschreibende und von anderen, nicht zu berücksichtigenden Metallobjekten (6) unterscheidende Hintergrundinformation bereitgestellt wird, und
- bei Verwendung einer Auswertungsfunktion für wenigstens ein Scoutbild (1) die Auswertungsfunktion die Hintergrundinformation als Eingangsdaten berücksichtigt und/oder mehrere auf unterschiedliche Metallobjekte (2, 6), insbesondere unterschiedliche Objektklassen, bezogene Auswertungsfunktionen bereitgestellt werden, von denen eine aufgrund der Hintergrundinformation zur Verwendung ausgewählt wird, und/oder
- beider wenigstens teilweisen Ermittlung der Objektinformation aus Navigationsdaten die Hintergrundinformation zur Auswahl zu verwendender Navigationsdaten verwendet werden.

11. Verfahren nach einem der vorangehenden Ansprüche, **dadurch gekennzeichnet, dass**, insbesondere durch die Auswertungsfunktion, als Objektinformation für jedes artefaktrelevante Metallobjekt (2) eine Objektklasse und/oder wenigstens zwei ausgezeichnete Referenzpunkte des Metallobjekts (2) und/oder wenigstens eine Bounding Box um das Metallobjekt (2) ermittelt wird.

12. Verfahren nach einem der vorangehenden Ansprüche, **dadurch gekennzeichnet, dass** die artefaktreduzierende Aufnahmetrajektorie als eine um einen optimierbaren Kippwinkel gekippte Kreistrajektorie ermittelt wird.

13. Röntgeneinrichtung (12), aufweisend eine Steuereinrichtung (16) und eine Aufnahmeanordnung zur Aufnahme von zweidimensionalen Projektionsbildern in verschiedenen Projektionsgeometrien entlang einer Aufnahmetrajektorie, aus denen ein dreidimensionaler Bilddatensatz rekonstruierbar ist, wobei die Aufnahmeanordnung einen Röntgenstrahler (14) und einen Röntgendetektor (15) umfasst, wobei die Steuereinrichtung (16) zur Durchführung eines Verfahrens nach einem der vorangehenden Ansprüche eingerichtet ist.

14. Computerprogramm, welches, wenn es auf einer Steuereinrichtung (16) einer Röntgeneinrichtung (12) ausgeführt wird, diese veranlasst, die Schritte eines Verfahrens nach einem der Ansprüche 1 bis 12 durchzuführen.

15. Elektronisch lesbarer Datenträger, auf dem ein Computerprogramm nach Anspruch 14 gespeichert ist.
